# EUROPEAN PATENT APPLICATION

(11) **EP 2 000 129 A2**
(43) Date of publication of application: **10.12.2008**
(21) Application number: 08251966.1
(22) Date of filing: 06.06.2008
(51) Int. Cl.: A61K 8/895, A61Q 5/06

(54) **Hair styling compositions containing a combination of a propylphenylsilsesquioxane resin and a phenylsilsesquioxane resin**

(30) Priority: 06.06.2007 US 942354 P
(71) Applicant: L'Oreal, 75008 Paris (FR)
(72) Inventor: Bui, Hy Si, Piscataway, NJ 08854 (US); Mecca, Jaimie, Clark, NJ 07066 (US); Castillo-Bucci, Carmen, Englewood, NJ 07631 (US)
(74) Representative: Srinivasan, Ravi Chandran

(57) **Abstract**

A process of styling hair involving applying onto the hair a composition containing: (a) at least one propylphenylsilsesquioxane resin comprising from about 30 to about 50 mole % of propyl siloxy units, based on the total mole % of siloxy units of the resin, and from about 50 to about 70 mole % of phenyl siloxy units, based on the total mole % of siloxy units of the resin; (b) at least one phenylsilsesquioxane resin; (c) at least one volatile solvent; and (d) optionally, at least one cosmetically-acceptable aromatic solvent.

## Description

This application is based on and claims the benefit of U.S. Provisional Application Serial No. 60/942,354, entitled HAIR STYLING COMPOSITIONS CONTAINING A COMBINATION OF A PROPYLPHENYLSILSESQUIOXANE RESIN AND A PHENYLSILSESQUIOXANE RESIN, filed June 6, 2007, the entire disclosure of which is incorporated by reference herein.

### BACKGROUND OF THE INVENTION

The present invention generally relates to a composition and process for styling hair that facilitates improved shaping and re-shaping of hair in a less tacky manner while imparting enhanced shine in order to make hair appear healthy and cared for.

Conventional hair styling products capable of shaping and re-shaping hair suffer from various disadvantages. First, such products tend to impart an exceptionally tacky feel onto the hair.

Thus, one object of the present invention is to provide a method of shaping and re-shaping hair in a manner which feels less tacky to the touch.

Another disadvantage associated with such products is that they tend to be easily removed when exposed to water and hair cleaning products.

Thus, it is another object of the present invention to provide a method of shaping and re-shaping hair capable of withstanding exposure to water and/or shampoo, thereby avoiding the need to have to be reapplied in order to provide additional hold.

Yet another disadvantage associated with conventional shaping/re-shaping products is the limited amount of shine they tend to impart onto hair.

Thus, it is another object of the present invention to provide a process for styling hair in a manner which is long lasting and imparts shine onto the hair with little, if any, tackiness.

In accordance with the invention, the term "hair styling composition" relates to any kind of hair composition that can be used to effect hair styling, for example, fixing compositions, setting compositions, permanent waving compositions, and hair treatment products.

### BRIEF SUMMARY OF THE INVENTION

The present invention is directed to a process for styling hair involving applying onto the hair a composition containing: (a) at least one propylphenylsilsesquioxane resin comprising from about 30 to about 50 mole % of propyl siloxy units, based on the total mole % of siloxy units of the resin, and from about 50 to about 70 mole % of phenyl siloxy units, based on the total mole % of siloxy units of the resin; (b) at least one phenylsilsesquioxane resin comprising about 100 mole % of phenyl siloxy units, based on the total mole % of siloxy units of the resin; (c) at least one volatile solvent; and (d) optionally, at least one cosmetically-acceptable aromatic solvent, wherein the propylphenylsilsesquioxane resin has a weight average molecular weight of from about 2,000 to about 30,000.

### DETAILED DESCRIPTION OF THE INVENTION

Other than in the operating examples, or where otherwise indicated, all numbers expressing quantities of ingredients and/or reaction conditions are to be understood as being modified in all instances by the term "about".

It has been surprisingly discovered that the combination of a propylphenylsilsesquioxane resin with a phenylsilsesquioxane resin eliminates the need for using conventional plasticizers in order to impart flexibility of the product on the hair.

Consequently, in a preferred embodiment of the present invention, the composition is substantially free of a plasticizer, i.e., contains less than about 0.5% by weight, such as less than about 0.25% by weight, and such as less than about 0.1% by weight, all weights based on the weight of the composition as a whole.

PROPYLPHENYLSILSESQUIOXANE RESIN

Silsesquioxane resins are a specific form of silicone resins. Silicone resins are crosslinked organopolysiloxanes which are solid at room temperature and generally soluble in organic solvents. When they are soluble in volatile solvents, silicone resins are capable of forming a film once the solvent has evaporated. Furthermore, if the solvent dissolving the silicone resin is absorbed on the substrate onto which it is applied, the silicone resin which remains on the substrate may also form a film.

The compositions of the present invention comprise propylphenylsilsesquioxane resins, which have been disclosed in patent publications WO2005/090444, published on September 29, 2005; US20040180011, published on September 16, 2004; and US20040156806, published on August 12, 2004, the entire contents of each of which are hereby incorporated by reference.

The propylphenylsilsesquioxane resin comprises propyl siloxy units (C₃H₇SiO_{3/2}) and phenyl siloxy units (C₆H₅SiO_{3/2}). The propyl siloxy units comprise from about 30 to about 50 mole % of the total mole % of siloxy units of the resin, and the phenyl siloxy units comprise from about 50 to about 70 mole % of the total mole % of siloxy units of the resin.

The propylphenylsilsesquioxane resin will have a weight average molecular weight of from about 2,000 to about 30,000, such as from about 3,000 to about 20,000.

The propylphenylsilsesquioxane resins preferably soften in the range of from about 30°C. to about 100°C., such as from about 30°C. to about 80°C., and such as from about 40°C. to about 70°C., as determined by DIN 53180 "Softening Point of Resins".

The mole % of propyl siloxy units to phenyl siloxy units can be adjusted depending on an intended application. As such, it is possible to have propylphenylsilsesquioxane resins having a mole % ratio of propyl siloxy units:phenyl siloxy units ranging from about 30:70 to about 50:50, such as 40:60; 50:50, and subranges therebetween.

A suitable example of a propylphenylsilsesquioxane resin for use in the composition of the present invention includes, but is not limited to, DC Z-6018, a resin having about 30 mole % of propyl siloxy units, and about 70 mole % of phenyl siloxy units, commercially available from Dow Corning.

The propylphenylsilsesquioxane resin is present in the composition in an amount ranging from about 3 to about 70% by weight, such as from about 3 to about 40% by weight, and such as from about 3 to about 25% by weight, all weights based on the weight of the composition as a whole.

PHENYLSILSESQUIOXANE RESIN

The phenylsilsesquioxane resin of the present invention is comprised of about 100 mole % of phenyl siloxy units. The phenylsilsesquioxane resin has a weight average molecular weight of from about 1,000 to about 10,000.

A suitable example of a phenylsilsesquioxane resin for use in the composition of the present invention includes, but is not limited to, DC 217, commercially available from Dow-Corning.

The phenylsilsesquioxane resin is present in the composition in an amount ranging from about 3 to about 70% by weight, such as from about 3 to about 40% by weight, and such as from about 3 to about 25% by weight, all weights based on the weight of the composition as a whole.

The weight ratio of the at least one propylphenylsilsesquioxane to the at least one phenylsilsesquioxane is dependent on the intended application. In general, the weight ratio will range from about 1:10 to about 10:1, such as about 1:5 to about 5:1; such as about 2:1 to about 1:2.

VOLATILE SOLVENTS

The compositions of the present invention comprise at least one volatile solvent.

The expression "volatile solvent" means any nonaqueous medium capable of evaporating from the keratinous material (i.e. hair or skin), at room temperature. Volatile solvents in particular comprise oils with a vapor pressure at room temperature and at atmospheric pressure ranging from 0.13 Pa to 40,000 Pa (10⁻³ to 300 mm Hg). These volatile oils especially facilitate the application of the composition to the keratinous material. These oils include, but are not limited to, silicone oils (optionally comprising alkyl or alkoxy groups that are pendant or at the end of a silicone chain), hydrocarbon-based oils, and fluoro oils, and mixtures thereof.

Suitable volatile silicone oils include, but are not limited to, linear or cyclic silicones containing from 2 to 7 silicon atoms, these silicones optionally comprising alkyl or alkoxy groups containing from 1 to 10 carbon atoms. Mention may thus be made especially of octamethylcyclotetrasiloxane, decamethylcyclopentasiloxane, hexadecamethylcyclohexasiloxane, heptamethylhexyltrisiloxane and heptamethyloctyltrisiloxane, and mixtures thereof.

Suitable volatile hydrocarbon-based oils include, but are not limited to, C₈-C₁₆ isoparaffins such as the Isopar and Permethyl products, and especially isododecane, isooctane, isodecane and isohexadecane, and mixtures thereof.

Other suitable volatile solvents include, but are not limited to, alcohols such as ethyl alcohol, isopropyl alcohol, benzyl alcohol and phenylethyl alcohol; glycol ethers such as, for example, ethylene glycol monomethyl, monoethyl or monobutyl ether, propylene glycol monomethyl ether, diethylene glycol alkyl ethers such as diethylene glycol monoethyl ether or monobutyl ether; and mixtures thereof.

The volatile solvent will typically be present in the composition of the present invention in an amount of from about 5 to about 90% by weight, such as from about 10 to about 80% by weight, such as from about 15 to about 70% by weight, all weights based on the weight of the composition as a whole.

COSMETICALLY-ACCEPTABLE AROMATIC SOLVENT

The composition of the present invention may also comprise a cosmetically-acceptable aromatic solvent.

The cosmetically acceptable aromatic solvent will comprise an aromatic portion in its molecule. Suitable cosmetically acceptable aromatic solvents include, but are not limited to, phenylated silicones, benzoate esters, benzyl esters, alkylated benzyl esters, alkylated benzyl ethers, and mixtures thereof. Preferred cosmetically acceptable aromatic solvents include, but are not limited to, trimethyl pentaphenyl trisiloxane available as DC555 from Dow Corning, C12-15 alkyl benzoate available as Finnsolv TN, PPG-3 benzyl ether myristate, available as Crodamol STS, PEG-2 benzyl ether available as Nikkol BZ-2, benzyl dodecanoate available as Pelemol 612, isostearyl benzoate available as Finsolv SB, phenethyl benzoate available as X-tend 226, 2-ethyl hexyl benzoate available as Finsolv EB, octyldodecyl benzoate available as Finsolv BOD, poly(propylene glycol) dibenzoates such as Dipropylene Glycol Dibenzoate, available as Finsolv PG-22.

The cosmetically-acceptable aromatic solvent may be present in the composition of the present invention in an amount of from about 0 to about 20% by weight, such as from about 0 to about 10% by weight, such as from about 0 to about 5% by weight, all weights based on the weight of the composition as a whole.

ADDITIVES/AUXILIARY AGENTS

The composition of the present invention may contain additional additives or auxiliary agents in order to provide additional cosmetic benefits and/or to facilitate various physical forms of said composition.

Fixative polymers chosen from anionic, cationic, amphoteric and non-ionic polymers, as well as mixtures thereof may be employed. The fixative polymer may additionally be halogenated, in particular fluorinated.

Emulsifying agents which can be used include those having an HLB of less than 7 and in particular fatty acid esters of polyols such as mono-, di-, tri- or sesqui-oleates or -stearates of sorbitol or glycerol, laurates of glycerol or polyethylene glycol; alkyl or alkoxy dimethicone copolyols having an alkyl or alkoxy chain pendant or at the end of a silicone-based backbone having for example from 6 to 22 carbon atoms. The emulsifying agents may also be those having an HLB greater than 7 such as fatty acid esters of polyethylene glycol (monostearate or monolaurate of polyethylene glycol); esters of fatty acids (stearate, oleate) of sorbitol which are polyoxyethylenated; polyoxyethylenated alkyl (lauryl, cetyl, stearyl, octyl) ethers and dimethicone copolyols. In general, it is possible to use nonionic or anionic or cationic emulsifiers well known to persons skilled in the art.

The styling composition of the present invention can also comprise hair-conditioning agents. Conditioning agents can be selected from oily substances, silicones, non-ionic substances, cationic amphiphilic ingredients, cationic polymers or their mixtures.

The hair styling composition of the present invention may also comprise additives, for instance those chosen from the non-exhaustive list which includes reducing agents, antioxidants, sequestering agents, softeners, antifoams, moisturizers, emollients, basifying agents, gelling agents, wetting agents, thickening agents, spreading agents, dispersants, plasticizers, sunscreens, direct dyes or oxidation dyes, pigments, mineral fillers, clays, colloidal minerals, nacres, nacreous agents, fragrances, peptizers, preserving agents, non-fixing polymers, ceramides, proteins, active agents, vitamins, antidandruff agents, and the like.

### EXAMPLE

The present invention will be better understood from the following examples, all of which are intended for illustrative purposes only, and are not meant to unduly limit the scope of the invention in any way.

The following examples are prepared as follows. The silsesquioxane resins are added to the aromatic solvent and blended for about 30 minutes. The volatile solvent is added and the mixing continued for about 15 minutes or until all the solids are dissolved.

About 0.25 cc of the prepared samples were applied to different clean and dried hair tresses with a pipette. The sample was distributed quickly with hands and combed through for even distribution. The samples were allowed to air-dry. The tresses were evaluated for flexibility, hold, shine, tack, distribution and playtime. This evaluation was performed visually and by touch. The amount of residue/flaking was evaluated visually after combing the treated tresses.

Each hair tress, onto which a hair styling composition was applied, was washed one time with Matrix Alternate Action Clarifying Shampoo, and was dried with a blow-dryer on a low heat setting. The tresses were then evaluated again for flexibility, hold, shine, tack, distribution and playtime. The evaluation was performed visually and by touch. The tresses were also combed at this point to evaluate the ease of comb.

Example 1: Inventive composition

| Ingredient (INCI Name) | w/w% |
|---|---|
| Propylphenylsilsesquioxane resin (DC Z-6018) | 13.0 |
| Phenylsilsesquioxane resin (DC217) | 13.0 |
| C12-15 Alkyl Benzoate | 2.6 |
| Denatured Alcohol | 71.4 |
| TOTAL | 100.0 |

Example 2: Comparative composition

| Ingredient (INCI Name) | w/w% |
|---|---|
| Trimethylsiloxysilicate (GE SR 1000) | 26.0 |
| C12-15 Alkyl Benzoate | 2.6 |
| Denatured Alcohol | 71.4 |
| TOTAL | 100.0 |

Hair tresses, to which the inventive composition was applied, exhibited shine and flexibility. They possessed the unique ability to mold a curl and to retain it.

After shampooing, the tresses still maintained the ability to hold a curl. The comparative composition, on the other hand, was brittle and flaked upon combing.

Example 3. Inventive composition

| Ingredient (INCI Name) | w/w% |
|---|---|
| Phenylpropylsilsesquioxane (50:50 Phenyl:Propyl) | 13.0 |
| Phenylsilsesquioxane (DC 217) | 13.0 |
| C12-15 Alkyl Benzoate | 2.6 |
| Denatured Alcohol | 71.4 |
| TOTAL | 100.0 |

Hair tresses treated with this composition exhibited shine and a slight tackiness, and had the ability to form a curl and to retain it. The tackiness was reduced after the shampooing step.

Example 4. Inventive composition

| Ingredient (INCI Name) | w/w% |
|---|---|
| Phenylpropylsilsesquioxane (70:30 Phenyl:Propyl) | 13.0 |
| Phenylpropylsilsesquioxane (50:50 Phenyl:Propyl) | 13.0 |
| C12-15 Alkyl Benzoate | 2.6 |
| Denatured Alcohol | 71.4 |
| TOTAL | 100.0 |

Hair tresses treated with this composition exhibited shine and a slight tackiness, and had the ability to form a curl and to retain it. The tackiness was reduced after the shampooing step.

It is to be understood that the foregoing describes preferred embodiments of the invention and that modifications may be made therein without departing from the spirit or scope of the invention as set forth in the claims.

## Claims

1. A process for styling hair comprising applying onto the hair a composition containing:
(a) at least one propylphenylsilsesquioxane resin having a weight average molecular weight of from about 2,000 to about 30,000, wherein the propylphenylsilsesquioxane resin comprises propyl siloxy units (C₃H₇SiO_{3/2}) and phenyl siloxy units (C₆H₅SiO_{3/2}), and wherein the propyl siloxy units comprise from about 30 to about 50 mole % of the total mole % of siloxy units of the resin, and the phenyl siloxy units comprise from about 50 to about 70 mole % of the total mole % of siloxy units of the resin;
(b) at least one phenylsilsesquioxane resin;
(c) at least one volatile solvent; and
(d) optionally, at least one cosmetically-acceptable aromatic solvent.

2. The process of claim 1, wherein (a) is present in the composition in an amount of from about 3 to about 70% by weight, based on the weight of the composition as a whole.

3. The process of claim 1 or 2, wherein (a) is present in the composition in an amount of from about 3 to about 25% by weight, based on the weight of the composition as a whole.

4. The process of any one of the preceeding claims, wherein (a) has weight average molecular weight from about 3,000 to about 20,000.

5. The process of any one of the preceeding claims, wherein (b) is present in the composition in an amount of from about 3 to about 70% by weight, based on the weight of the composition as a whole.

6. The process of claim 5, wherein (b) is present in the composition in an amount of from about 3 to about 25% by weight, based on the weight of the composition as a whole.

7. The process of any one of the preceeding claims, wherein (a) and (b) are present at a weight ratio ranging from about 1:10 to about 10:1.

8. The process of any one of the preceeding claims, wherein (c) is present in the composition in an amount of from about 5 to about 90% by weight, based on the weight of the composition as a whole.

9. The process of any one of the preceeding claims, wherein (c) is a mixture of a volatile hydrocarbon solvent and an alcohol.

10. The process of any one of the preceeding claims, wherein (d) is C12-15 alkyl benzoate.

11. The process of any one of the preceeding claims, wherein the composition is substantially free of a plasticizer.
